# EUROPEAN PATENT SPECIFICATION

(11) **EP 0 964 713 B1**
(45) Date of publication and mention of the grant of the patent: **15.09.2004**
(21) Application number: 97947813.8
(22) Date of filing: 10.12.1997
(51) Int. Cl.: A61M 16/04

(54) **DEVICE FOR CONTROLLING THE PRESSURE OF AN ENDOTRACHEAL CUFF**
VORRICHTUNG ZUR KONTROLLE DES DRUCKES IN DER DICHTMANSCHETTE EINES ENDOTRACHEALTUBUS
DISPOSITIF SERVANT A REGULER LA PRESSION D'UN BALLONNET TRACHEAL

(30) Priority: 10.12.1996 GB 9625590
(43) Date of publication of application: 22.12.1999
(73) Proprietor: Kamath, Belady Sumanth Kumar, London NW4 4HG (GB); Abdelatti, Mohamed Osman, Rochdale OL12 9QB (GB)
(72) Inventor: Kamath, Belady Sumanth Kumar, London NW4 4HG (GB); Abdelatti, Mohamed Osman, Rochdale OL12 9QB (GB)
(74) Representative: Smith, Norman Ian
(86) International application number: PCT/GB1997/003403
(87) International publication number: WO 1998/025663

(56) References cited:
- DE-A- 2 055 049
- DE-A- 3 919 634
- US-A- 4 298 000
- US-A- 4 608 042
- US-A- 5 209 731

## Description

The present invention relates to apparatus for use in carrying out anaesthetic and similar routines.

Generally, when carrying out an anaesthetic or similar routine in an operating theatre or intensive care unit, an endotracheal tube is inserted through the mouth or occasionally the nose and into the trachea (windpipe) of the patient, for supply of Oxygen and/or a mixture of anaesthetic gases with Oxygen to the lungs.

Normally the endotracheal tube incorporates an inflatable cuff near its lower end from which an air inflatable pilot tube leads out to a pilot balloon and a non-return valve. The cuff surrounds the endotracheal tube and should be inflated to a pressure which then seals off the trachea passageway surrounding the endotracheal tube and thereby ensures that liquid or other material which might come up from the stomach or elsewhere during an operation cannot enter the lungs.

In use, the air inflation pilot tube is inflated to a desired pressure with a hand syringe and the anaesthetist has to ensure, by observation of the pilot balloon, that the inflation cuff is sealed to the required pressure, and also he has to carry out other duties at the same time relating to the supply of Oxygen and/or anaesthetic gases and general monitoring of the patient's breathing and other life functions.

US 5,209,731 discloses a handheld gun for inflating and aspirating large volume balloons which are suitable for use in medical procedures. The handheld gun has a handle and trigger arrangement for controlling movement of a plunger in a syringe to inflate and/or deflate a balloon. Operation of the trigger when the plunger is depressed within the cylinder causes rapid deflation of the balloon.

The present invention is concerned with solving the problem of facilitating inflation of the endotracheal tube inflation cuff.

Accordingly, the invention provides an apparatus for the application of pressure to an inflatable cuff of an endotracheal tube via an air pressure syringe comprising:
a pressure storage means,
means for applying pressure to load said pressure storage means,
a releasable catch means,
and connection means for operative connection of said apparatus via an air syringe, to an inflatable cuff, characterised in that
the releasable catch means is a releasable catch means for releasably holding said pressure storage means in a loaded condition, and wherein the apparatus is arranged so that on release of the catch the pressure storage means acts on an air syringe via which the apparatus is connected to an inflatable cuff to supply air from the syringe into the inflatable cuff,

The apparatus may include an air pressure syringe, or the apparatus may be supplied as a unit for attachment to an existing air pressure syringe.

The term air pressure syringe in this specification means a pressure applying instrument or device, such as a piston and cylinder arrangement, usually but not necessarily manually operable, and arranged to apply air pressure at its outlet. An alternative form of syringe may be based on use of collapsible bellows rather than a piston and cylinder.

The pressure storage means may comprise a pair of tubes, one of which slidingly fits inside the other and an energy storage spring, e.g. a compressible coil spring located to hold the tubes apart. Alternatively there may be only a single tube with the spring located in its interior. Then, a protruding handle may be provided which draws the tube or one of the two tubes forward against spring pressure, preferably the spring acting in compression, to load the device.

The pair of tubes may form part of an air pressure syringe or may be provided as separate components from the air pressure syringe.

A simple form of releasable catch means may be provided which operates in co-operation with the protruding handle. The protruding handle can be provided with one or more slots, and the catch means may be arranged to engage in a slot to hold the releasable catch means in a loaded condition, and to be released to permit the stored pressure energy to be applied to an air syringe. An important advantage of this arrangement is that the assistant to the anaesthetist then has a hand released to be usable for other functions.

In an alternative form of releasable catch means, a protruding handle carried on a movable end of the storage means protrudes into a longitudinal slot in a housing of the apparatus and can be engaged at different locations in lateral slots leading off the longitudinal slot, to hold the pressure storage means in a loaded condition.

The connection means may be a slotted seat into which the plunger handle of an air syringe may be located. Generally a housing will be provided which locates and carries the pressure storage means and a plunger when they are operatively connected. The strength of spring will be selected to provide the required pressure to the air syringe.

Generally an extension line, for example 100 cm in length, should be provided between the syringe and the endotracheal tube so that the apparatus can be located at a convenient position away from the principal area of activity during an operation.

The apparatus has a number of operational advantages. It enables the endotracheal tube cuff to be automatically and consistently inflated. This releases a hand of the anaesthetist's assistant for other functions; and also - particularly during emergency anaesthesia - allows for consistent prevention of inhalation by the patient of stomach contents and facilitates efficient ventilation of the lungs.

It enables the volume of air in the cuff to be increased or decreased in a controlled way and it can be arranged for continuous monitoring of the cuff volume.

An embodiment of the invention will now be described by way of example with reference to the accompanying diagrammatic drawings in which:-
Figure 1 shows insertion of an endotracheal tube into a patient;
Figure 2 shows a typical endotracheal tube;
Figure 3a shows a device in accordance with the invention;
Figure 3b shows an alternative form, of the device; and
Figure 4 shows an alternative form of release means;

It is standard practice to use an endotracheal tube when carrying out anaesthetic or similar routines where Oxygen and other gases have to be supplied to the lungs.

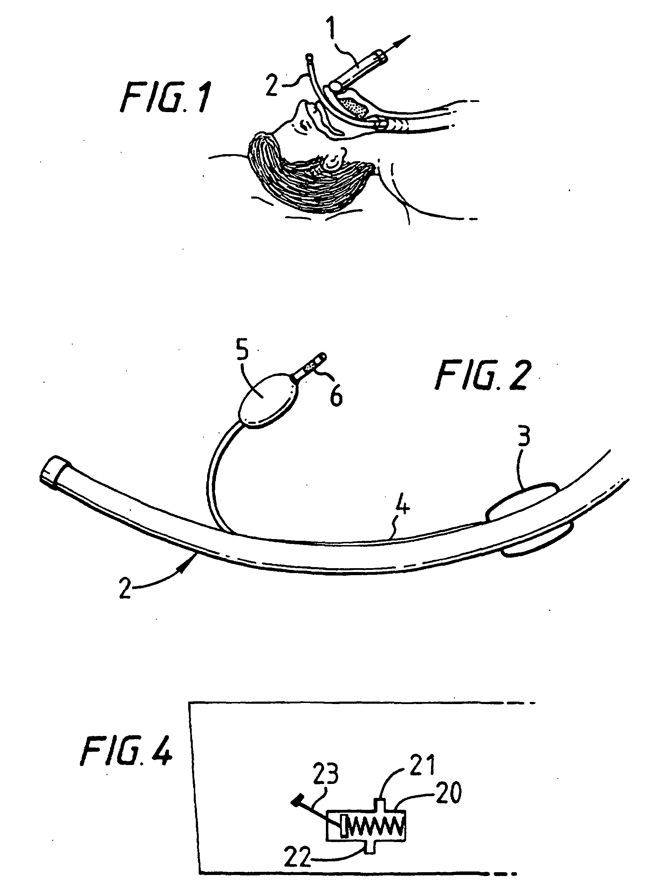

Figure 1, which comes straight out of a standard text book, shows that a laryngoscope 1 forces the tongue to the side of the mouth so that an endotracheal tube 2 can be inserted into the trachea.

Referring to Figure 2, endotracheal tubes of various sizes to suit various patients can be provided and the illustration shows a typical such tube which normally will, be disposable and has to be manufactured under sterile conditions, has an inflatable cuff 3 linked by a pilot tube 4 to a pilot balloon 5 and then terminates at an outlet connection incorporating a one-way valve 6.

An air pressure syringe is temporarily attached to the end of the pilot tube after the endotracheal tube has been inserted and the cuff is inflated while the pilot balloon is observed to ensure that a suitable degree of inflation has been achieved. Up to this point we are discussing known and quite standard arrangements.

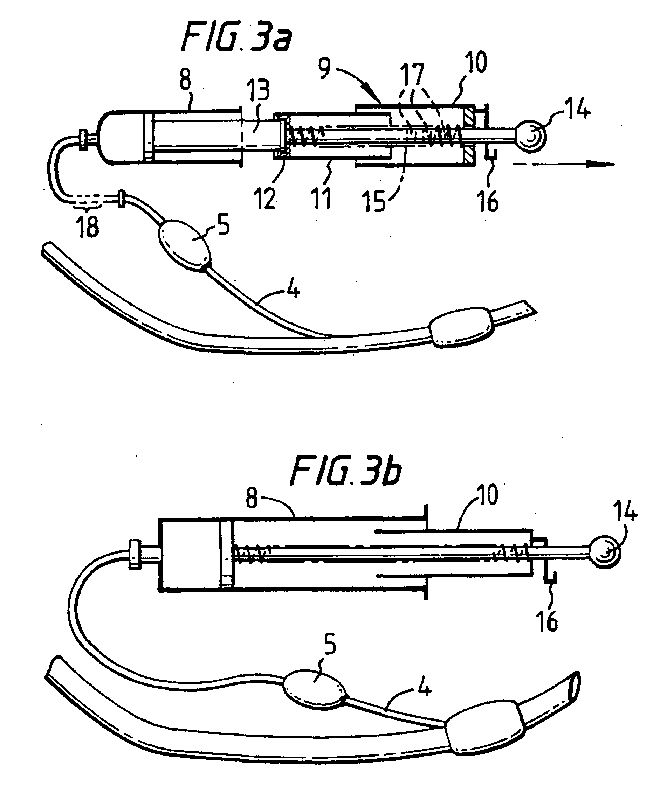

Referring to Figure 3a, which shows an apparatus in accordance with the invention for applying pressure to the air syringe, a location container (not shown) with slots or guides to maintain various components in position carries a standard syringe 8 of 20 ml capacity and a pressure storage device 9.

The location container is arranged so that standard air syringes of varying size can be accommodated. The pressure storage device 9 is formed essentially from two tubes 10 and 11 one of which slides within the other, the inner of the two cylindrical tubes 11 is movable and incorporates an end connection means 12 defining a slotted seat to receive the handle end of a standard syringe plunger 13, while the outer tube is maintained statically in position within the location container.

The cylinder 11 is arranged to be movable in an axial direction by means of a longitudinal draw handle 14 of stainless steel or other suitable material which is attached to the left hand end (as viewed in the Figure) of the device 9. A steel or other suitable material compression spring 15 is located around the handle 14, and constrains the device against inward movement and stores the pressure energy.

The outlet of the syringe 8 is connected via an extension line 18 some 100 cm in length to an endotracheal pilot tube 4.

In order to store pressure energy in the storage device 9, the draw handle 14 is pulled to the right as viewed in the figure which compresses the coil spring 15 and draws the plunger of the syringe 8 to the right. A latch 16 is provided which co-operates with slots 17 on the draw handle 14 so that when the draw handle is pulled out a predetermined distance the latch 16 simply drops into a slot 17 holding the draw handle in a loaded position. There are three slots 17 which are calibrated so that when the device is used with a 20 millilitre syringe, the first slot pre-loads for supply of 11 ml of air, the second for 13 ml and the third for 15 ml of air. The device can operate with other numbers of slots or with calibrations for other capacity syringes.

Then in use, and after loading the pressure storage device 9, it is merely necessary to release the catch 16 with the flick of a finger whereupon the draw handle 14 and the movable tube 11 will be forced to move to the left as viewed in the figure under the action of the spring 15 so as to supply compressed air from the syringe 8 into the pilot tube 4 of the endotracheal tube 2.

In an alternative form shown in Figure 3b only a single tube 10 is provided and is located within the tubular wall of the syringe 8 itself (which may be of 20ml capacity) . In other respects the device works in the same way in that a handle 14 is drawn back to load the spring and the catch 16 is released when required for actuation of the device. This example has an advantage in that it uses less components.

Figure 4 illustrates an alternative releasable catch means and shows a housing for the device which includes a longitudinal slot 20 and lateral slots 21 and 22. A releasable catch 23 in the form of a protruding lever attached to the moving end of the storage means, i.e. the tube 11, can be engaged in one or other of the lateral slots.

An optional system to regulate the endotracheal cuff pressure e.g. a low resistance cylinder and piston or a bellows arrangement supported by means of a spring can be incorporated with the device. This system can also be used independently for the same purpose of regulating the cuff pressure without being an integral part of the device.

## Claims

1. An apparatus for the application of pressure to an inflatable cuff (3) of an endotracheal tube via an air pressure syringe (8) comprising:
a pressure storage means (9),
means for applying pressure to load said pressure storage means (9),
a releasable catch means (16),
and connection means (12) for operative connection of said apparatus via an air syringe (8), to an inflatable cuff (3), **characterised in that**
the releasable catch means (16) is a releasable catch means (16) for releasably holding said pressure storage means (9) in a loaded condition, and wherein the apparatus is arranged so that on release of the catch (16) the pressure storage means (9) acts on an air syringe (8) via which the apparatus is connected to an inflatable cuff (3) to supply air from the syringe (8) into the inflatable cuff (3).

2. An apparatus according to claim 1 comprising a tubular storage arrangement (9) including a pair of tubes (10, 11), one of which slidingly fits inside the other and an energy storage spring (15) which is located to hold the tubes apart.

3. An apparatus according to claim 2 comprising a protruding handle (14) which is arranged to draw the two tubes (10, 11) together against spring pressure to load the device.

4. An apparatus according to claim 3 in which the releasable catch means (16) is arranged to operate in co-operation with the protruding handle (14) to allow release of stored pressure.

5. An apparatus according to claim 4 in which the protruding handle (14) is provided with one or more slots (17), and the catch means (16) is arranged to engage in a slot to hold the releasable catch means (16) in a loaded condition, and to be released to permit the stored pressure energy to be applied to an air syringe.

6. An apparatus according to claim 5 in which the connection means (12) is a slotted seat (12) into which the plunger handle of an air syringe may be located.

7. An apparatus according to any preceding claim comprising a housing which is arranged to locate and carry the pressure storage means (9) and a plunger when they are operatively connected.

8. An apparatus according to any preceding claim comprising an extension line (18), for example 100 cm in length, for provision between the syringe and the endotracheal tube so that the apparatus can be located at a convenient position away from the principal area of activity during an operation.

9. An apparatus according to any preceding claim comprising a pressure controlling system to regulate pressure within the cuff (3) which comprises a low resistance spring loaded piston or bellows arrangement.

10. An apparatus according to any preceding claim in combination with an endotracheal tube and arranged to apply pressure to an inflation cuff (3) of an endotrachial tube.

## Patentansprüche

1. Vorrichtung zur Anwendung von Druck auf eine aufblasbare Manschette (3) einer endotrachealen Röhre mittels einer Luftdruckspritze (8), wobei die Vorrichtung Folgendes umfasst:
ein Druckspeichermittel (9),
Mittel zur Anwendung von Druck, um das Druckspeichermittel (9) zu laden,
ein lösbares Sperrmittel (16),
und Verbindungsmittel (12) für eine betriebsfähige Verbindung der Vorrichtung mit einer aufblasbaren Manschette (3) mittels einer Luftspritze (8), **dadurch gekennzeichnet, dass**
das lösbare Sperrmittel (16) ein lösbares Sperrmittel (16) zum lösbaren Halten des Druckspeichermittels (9) in einem geladenen Zustand ist, und wobei die Vorrichtung so angeordnet ist, dass das Druckspeichermittel (9) bei Lösen der Sperre (16) auf eine Luftspritze (8) wirkt, mittels derer die Vorrichtung mit einer aufblasbaren Manschette (3) verbunden ist, um der aufblasbaren Manschette (3) Luft von der Spritze (8) zuzuführen.

2. Vorrichtung nach Anspruch 1, die eine röhrenförmige Speicheranordnung (9) umfasst, die ein Paar Röhren (10, 11), von denen eine gleitfähig in die andere passt, sowie eine Energiespeicherfeder (15) enthält, die so angeordnet ist, dass sie die Röhren voneinander beabstandet hält.

3. Vorrichtung nach Anspruch 2, die einen hervorstehenden Griff (14) umfasst, der so angeordnet ist, dass er die beiden Röhren (10, 11) gegen den Federdruck zusammenzieht, um die Vorrichtung zu laden.

4. Vorrichtung nach Anspruch 3, bei der das lösbare Sperrmittel (16) so angeordnet ist, dass es mit dem hervorstehenden Griff (14) zusammenwirkt, um das Ablassen von gespeichertem Druck zu ermöglichen.

5. Vorrichtung nach Anspruch 4, bei der der hervorstehende Griff (14) mit einem oder mehreren Schlitzen (17) versehen ist und das Sperrmittel (16) so angeordnet ist, dass es in einen Schlitz eingreift, um das lösbare Sperrmittel (16) in einem geladenen Zustand zu halten, und gelöst werden kann, so dass die gespeicherte Druckenergie auf eine Luftspritze angewendet wird.

6. Vorrichtung nach Anspruch 5, bei der das Verbindungsmittel (12) ein geschlitzter Sitz (12) ist, in dem der Tauchkolbengriff einer Luftspritze angeordnet werden kann.

7. Vorrichtung nach einem der vorhergehenden Ansprüche, die ein Gehäuse, das so angeordnet ist, dass es das Druckspeichermittel (9) positioniert und trägt, sowie einen Tauchkolben umfasst, wenn diese in betriebsfähiger Weise angeschlossen sind.

8. Vorrichtung nach einem der vorhergehenden Ansprüche, die eine Verlängerungsleitung (18) mit einer Länge von beispielsweise 100 cm umfasst, um zwischen der Spritze und der endotrachealen Röhre angeordnet zu werden, so dass die Vorrichtung während einer Operation entfernt vom Hauptaktivitätsbereich an einer angemessenen Stelle angeordnet werden kann.

9. Vorrichtung nach einem der vorhergehenden Ansprüche, die ein Druckregelsystem zur Regelung des Drucks in der Manschette (3) umfasst, wobei das System einen Kolben, der mit einer Feder mit geringem Widerstand vorgespannt ist, oder eine Blasebalganordnung umfasst.

10. Vorrichtung nach einem der vorhergehenden Ansprüche in Kombination mit einer endotrachealen Röhre, die so angeordnet ist, dass Druck auf eine Aufblasmanschette (3) einer endotrachealen Röhre angewendet werden kann.

## Revendications

1. Appareil pour appliquer une pression sur un manchon gonflable (3) d'un tube endotrachéal par l'intermédiaire d'une seringue à air comprimé (8) comprenant :
un moyen de stockage de pression (9),
un moyen pour appliquer une pression pour charger ledit moyen de stockage de pression (9),
un moyen d'enclenchement pouvant être déclenché (16), et un moyen de raccordement (12) pour raccorder de façon à ce qu'il puisse fonctionner ledit appareil, par l'intermédiaire d'une seringue à air (8), à un manchon gonflable (3), **caractérisé en ce que**
ledit moyen d'enclenchement pouvant être déclenché (16) est un moyen d'enclenchement pouvant être déclenché (16) pour maintenir, tout en pouvant être déclenché, ledit moyen de stockage de pression (9) dans un état chargé, et dans lequel l'appareil est disposé de façon à ce que, lors du déblocage du dispositif d'enclenchement (16), le moyen de stockage de pression (9) agit sur une seringue à air (8) par l'intermédiaire de laquelle l'appareil est raccordé à un manchon gonflable (3) pour alimenter de l'air depuis la seringue (8) jusque dans le manchon gonflable (3).

2. Appareil selon la revendication 1 comprenant un dispositif de stockage tubulaire (9) comprenant une paire de tubes (10, 11) dont un glisse à l'intérieur de l'autre et un ressort de stockage d'énergie (15) qui est positionné de façon à maintenir les tubes séparés.

3. Appareil selon la revendication 2 comprenant une poignée faisant saillie (14) qui est disposée de façon à tirer les deux tubes (10, 11) l'un vers l'autre contre la pression d'un ressort pour charger le dispositif.

4. Appareil selon la revendication 3 dans lequel le moyen d'enclenchement pouvant être déclenché (16) est disposé de façon à fonctionner en coopération avec la poignée faisant saillie (14) pour permettre le relâchement de la pression stockée.

5. Appareil selon la revendication 4 dans lequel la poignée faisant saillie (14) est pourvue d'une ou de plusieurs encoches (17), et le moyen d'enclenchement (16) est disposé de façon à s'engager dans une encoche pour maintenir le moyen d'enclenchement pouvant être déclenché (16) dans un état chargé et à être débloqué pour permettre à l'énergie de la pression stockée d'être appliquée sur une seringue à air.

6. Appareil selon la revendication 5 dans lequel le moyen de raccordement (12) est un siège à encoches (12) dans lequel la poignée du plongeur d'une seringue à air peut être placée.

7. Appareil selon l'une quelconque des revendications précédentes comprenant un logement qui est disposé de façon à positionner et à porter le moyen de stockage de pression (9) et un plongeur lorsqu'ils sont raccordés de façon à pouvoir fonctionner.

8. Appareil selon l'une quelconque des revendications précédentes comprenant une ligne de prolongement (18), par exemple d'une longueur de 100 cm, montée entre la seringue et le tube endotrachéal de façon à ce que l'appareil puisse être placé dans une position commode éloignée de la zone principale d'activité pendant une opération.

9. Appareil selon l'une quelconque des revendications précédentes comprenant un système de régulation de pression pour réguler la pression à l'intérieur du manchon (3) qui comprend un piston chargé par ressort à faible résistance ou un ensemble soufflet.

10. Appareil selon l'une quelconque des revendications précédentes en combinaison avec un tube endotrachéal et disposé de façon à appliquer une pression sur un manchon gonflable (3) d'un tube endotrachéal.
